# EUROPEAN PATENT APPLICATION

(11) **EP 1 985 276 A1**
(43) Date of publication of application: **29.10.2008**
(21) Application number: 07008528.7
(22) Date of filing: 26.04.2007
(51) Int. Cl.: A61H 1/02, A61K 38/48

(54) **Treatment of movement disorders by a combined use of a chemodenervating agent and automated movement therapy**

(71) Applicant: Merz Pharma GmbH & Co. KGaA, 60318 Frankfurt am Main (DE)
(72) Inventor: Grafe, Susanne, 60599 Frankfurt am Main (DE); Heinen, Florian Dr., 80805 München (DE); Berweck, Steffen Dr., 80805 München (DE); Borggräfe, Ingo Dr., 81667 München (DE)
(74) Representative: Ricker, Mathias

(57) **Abstract**

The present invention relates to a method of treating a movement disorder in a patient, the method comprising administering a medicament comprising an effective amount of chemodenervating agent to said patient, wherein said patient is subjected to an automated movement therapy, and said medicament is administered prior to and/or during and/or after said movement therapy and a kit for the treatment of patients suffering from movement disorders comprising a medicament comprising an effective amount of a chemodenervating agent, and a device for carrying out automated movement therapy.

## Description

### FIELD OF INVENTION

The present invention relates to methods for treating movement disorders by a combined use of a chemodenervating agent and automated movement therapy and a kit comprising said medicament and a device for performing automated movement therapy.

### BACKGROUND OF INVENTION

Locomotion therapy for regaining walking capacity using the principle of enhancing neuroplasticity by task specific training has been well established in the (re)habilitation process of patients with central gait disorders (Hesse S. (2001) Locomotor therapy in neurorehabilitation, NeuroRehabilitation 16: 133-139).

Conventional over-ground gait training (COGT) in adults has been endorsed by the method body weight supported treadmill training (BWSTT), thereby gaining functional benefits such as symmetry and increased walking speed (Barbeau H, Visintin M. (2003) Optimal outcomes obtained with body-weight support combined with treadmill training in stroke subjects, Arch Phys Med Rehabil84: 1458-1465; McNevin NH, Coraci L, Schafer J. (2000) Gait in adolescent cerebral palsy: the effect of partial unweighting, Arch Phys Med Rehabil81: 525-528). However, the assignment of human resources for manual assistance in these methods is considerable. Controlled trials of adult patients with traumatic brain injury (TBI) or incomplete spinal cord injury (SCI) have been conducted by using BWSTT and COGT (Dobkin B, Apple D, Barbeau H, Basso M, Behrman A, Deforge D, et al. (2006) Weight-supported treadmill vs over-ground training for walking after acute incomplete SCI, Neurology66: 484-493; Wilson DJ, Powell M, Gorham JL, Childers MK (2006) Ambulation training with and without partial weightbearing after traumatic brain injury: results of a randomized, controlled trial,Am J Phys Med Rehabil85: 68-74).

In children with cerebral palsy (CP), general functional-strength training has been found effective to improve functional performance. Promising evidence exists that intensive BWSTT can improve walking capacity in these children (Song WH SI, Kim YJ, Yoo JY, (2003) Treadmill training with partial body weight support in children with cerebral palsy, Arch Phys Med Rehabil84 (E2)).

In order to restore or develop walking abilities, repetition and intensity of training seems to be a crucial key to motor (re)learning. Thus, automated gait training devices have been developed during the last decade to further improve gait training (Colombo G, Joerg M, Schreier R, Dietz V. (2000) Treadmill training of paraplegic patients using a robotic orthosis, J Rehabil Res Dev37: 693-700; Hesse S, Schmidt H, Werner C, Bardeleben A (2003), Upper and lower extremity robotic devices for rehabilitation and for studying motor control, Curr Opin Neurol16: 705-710).

Such a device is described in detail in US 6,821,233. This patent relates to an automatic machine to be used in treadmill therapy of paraparetic and hemiparetic patients and which automatically guides the legs on the treadmill. The claimed machine consists of a driven and controlled orthotic device which guides the legs in a physiological pattern of movement, a treadmill and a relief mechanism. An improved relief mechanism, which helps to support the patient by unloading at least some of its body weight is the subject of EP 1 586 291. Further devices for locomotion therapy are the subject of US 6,059,506 and US 6,685,658, respectively.

Devices for automated locomotion therapy are commercially available from Hocoma AG, e.g. under the trademark Lokomat^{®}. A pediatric module of the DGO Lokomat^{®} has been developed very recently, which allows training of children starting at an age of approximately 4 years and older.

One specific aspect of the movement disorders that are to be treated according to the present invention is related to cerebral palsy (CP) in children. CP is the most frequent movement disorder in children. It occurs within 1.5 to 2.5 per 1000 children. CP is a disorder of the development of movement and appearance and is caused by damages within the young brain still to be developed (early brain lesion). As such, CP is a collective name given to a range of conditions caused by, e.g., early brain lesion caused before, at or around the time of birth, or in the first year of life. As used herein, CP also includes any other cause for diseases or disorders resulting in hyperactive muscles. The brain injury may be caused by a variety of conditions, e.g. by prematurity. Although the brain injury causing cerebral palsy is a non-progressive injury, its effects may change as the patient grows older. This may result in dynamic contractures of the muscles, which tend to change over time to fixed contractures and which impair or inhibit completely the patient's ability to use the affected muscles.

Besides the above-mentioned locomotion therapy, the method of choice for treating CP, in many cases traditionally was surgery. In recent years, botulinum toxin as a bacterial toxin has been used in the treatment of cerebral palsy. An overview of the treatment of CP with botulinum toxin may be found in European patent EP 0 605 501 as well as in the "European Consensus Table 2006 on botulinum toxin for children with cerebral palsy", European Journal of Pediatric Neurology 10 (2006), 215-225 and the literature cited therein.

In view of the above-cited prior art, it is an object of the present invention to provide an alternative treatment of movement disorders, e.g. for the treatment of movement disorders occurring in conjunction with CP in children. It is a further object of the present invention to provide an improved therapy of these disorders that is preferably related to individual motor development.

It is still another object to provide a kit specifically designed for a patient suffering from movement disorders of the kind to be treated herein.

### SUMMARY OF THE INVENTION

The above and other objects are solved by the use of an effective amount of a chemodenervating agent for the manufacture of a medicament for administering to a patient for treating a movement disorder in said patient, wherein said patient is subjected to an automated movement therapy, and wherein said medicament is administered prior to and/or during and/or after said movement therapy.

Furthermore, the present invention relates to a kit for the treatment of patients suffering from movement disorders comprising a medicament comprising an effective amount of a chemodenervating agent and a device for carrying out automated movement therapy.

### DETAILED DESCRIPTION OF THE INVENTION

The patient to be treated by the present method and kit can be of animal or human nature. Preferably, the patient is human. More preferably, the described and claimed treatment is for a young patient, especially in regard to movement disorders associated with cerebral palsy. In this respect, the term "young" refers to a patient that has not completed its motor development and fixed muscle contractures have not occurred. In another embodiment, the patient can be a child of up to 6-8 years in age with unfinished motor development and motor maturation. Preferably, the children to be treated are up to six years old.

In another embodiment, the treatment of movement disorders involves symptoms of the underlying condition, e.g. CP, in particular symptoms of include difficulties with fine motor tasks (such as writing or using scissors), difficulty maintaining balance or involuntary movements. The symptoms may differ from person to person and may change over time.

In still another embodiment, the movement disorder is a hyperkinetic and/or hypokinetic movement disorder, wherein an imbalance between agonist and antagonist is interfering with muscle function.

In one embodiment of the present invention, the movement disorder involves spasticity of a muscle. In another embodiment of the present invention, the spasticity is, or is associated with, post-stroke spasticity or a spasticity caused by cerebral palsy.

"Spasticity" is defined as a motor disorder characterized by a velocity-dependent increase in tonic stretch reflexes (muscle tone) with exaggerated tendon jerks, resulting from hyperexcitability of the stretch reflex, as one component of the upper motor neuron syndrome. In some patients spasticity can be beneficial, as in the case of hip and knee extensor spasticity, which may allow weight bearing, with the affected limb acting like a splint. However, in the majority of patients spasticity causes difficulties with activities of daily living, such as dressing and cleaning the palm of the clenched hand. Some of the spasticity conditions that may involve movement disorders to be treated according to the present invention are exemplarily given in Table 1 below:

**Table 1: Spasticity Conditions**

| | | |
|---|---|---|
| ➢ in encephalitis and | ■ autoimmune processes | ○ multiple sclerosis |
| myelitis | | ○ transverse myelitis |
| | | ○ devic syndrome |
| | ■ viral infections | |
| | ■ bacterial infections | |
| | ■ parasitic infections | |
| | ■ fungal infections | |
| ➢ in hereditary spastic paraparesis | | |
| ➢ in central nervous system hemorrhage | ■ intracerebral hemorrhage | |
| | ■ subarachnoidal hemorrhage | |
| | ■ subdural hemorrhage | |
| | ■ intraspinal hemorrhage | |
| | | |
| ➢ in neoplasias | ■ hemispheric tumors | |
| | ■ brainstem tumors | |
| | ■ myelon tumors | |

The term "post-stroke spasticity" relates to spasticity occurring after a stroke incident. Stroke is a leading cause of long-term disability, with spasticity occurring in 19% to 38% of patients (Watkins CL, Leathley MJ, Gregson JM, Moore AP, Smith TL, Sharma AK, Prevalence of spasticity post stroke, Clin Rehabil2002; 16(5): 515-522. (ID 1915001)).

Cerebral palsy is an umbrella-like term used to describe a group of chronic disorders impairing control of movement that appear in the first years of life and generally do not worsen over time. The disorders are caused by faulty development or damage to motor areas in the brain that disrupts the brain's ability to control movement and posture.

It is our understanding that cerebral palsy cannot be treated, i.e. that the injury to the brain cannot be undone. Instead, it is intended to treat some symptoms of cerebral palsy, in particular those related to movement disorders. These symptoms are caused by an abnormal or disturbed muscle activity which prevents the affected muscles from relaxing. "Cerebral palsy" describes a wide spectrum of pyramidal dysfunctions causing paresis, extrapyramidal dysfunctions causing dystonia, rigidity, spasticity and spasms, apraxic components and coordinative dysfunctions. Cerebral palsy (Koman LA, Mooney JF, Smith BP, Goodman A, Mulvaney T. Management of spasticity in cerebral palsy with botulinum - A toxin: report of a preliminary, randomized, double-blind trial, J Pediatr Orthop 1994; 14(3): 299-303. (ID 1767458); Pidcock FS, The emerging role of therapeutic botulinum toxin in the treatment of cerebral palsy, J Pediatry2004; 145(2 Suppl): S33-S35. (ID 2994781)) may occur after brain hemorrhage, asphyxia, premature birth or other perinatal complications. It is a life-long condition causing uncoordinated movements, paresis and various forms of muscle hyperactivity. Patients affected by cerebral palsy, when treated in accordance with the methods disclosed herein, experience a functional improvement of hyperactive muscles. It is, however, well within the scope of the present invention to improve muscle activity of muscles not affected by spasticity. This also includes coordination between different muscle groups. The term "muscle groups" includes adjacent muscles but also muscles in different body regions.

In accordance with the teaching of the present invention, common clinical patterns of movement disorders associated with spasticity in the corresponding muscle groups or movement disorders associated with cerebral palsy are treated by the method according to the invention, i.e. a combination of locomotion/movement therapy and administration of botulinum toxin.

In particular, the movement disorder is associated with cerebral palsy, M. Parkinson, central gait impairment, spinal cord injuries, dystonias, traumatic brain injury, genetic disorders, metabolic disorders, dynamic muscle contractures and/or stroke are treated, e.g. movement disorders which are associated with at least one selected among pes equinus, pes varus, lower limb spasticity, upper limb spasticity, adductor spasticity, hip flexion contracture, hip adduction, knee flexion spasticity (crouch gait), plantar flexion of the ankle, supination and pronation of the subtalar joint, writer's cramp, musician's cramp, golfer's cramp, leg dystonia, thigh adduction, thigh abduction, knee flexion, knee extention, equinovarus deformity, foot dystonia, striatal toe, toe flexion, toe extension.

One part of the treatment as claimed is the so-called automated movement therapy. The term "movement therapy" relates to any kind of therapy, wherein the patient is trained to move its extremities in a "normal" manner, i.e. in a physiological movement or a sequence of movements. The therapy of upper and lower extremities shall be included in the term "movement therapy". The term "automated movement therapy" relates to any kind of movement therapy wherein the patient is trained to use its muscles in a "normal" manner, i.e. a physiological (sequence of) movement(s), by way of an automated (driven) orthotic device. The movement therapy being part of the present invention encompasses, but is not limited to, the selective movement of upper and lower extremities and/or parts thereof, such as arms and legs as well as the shoulder, elbow, wrist, hand, finger, thumb, knee, feet, and toe joints in multiple ways, either isolated or within movement chains.

In general, a device which is to be used within the method and the kit of the present invention comprises a driven and controlled orthotic device, which guides the extremities, e.g. the legs or arms of the patient, in a physiological pattern of movement. Preferably, the device is supported by an automated gait orthosis or an arm mover.

For patients having problems with their leg movement, the device preferably comprises an automated gait orthosis, more preferably in combination with a treadmill. Respective devices for an automated movement (locomotion) therapy are meanwhile commercially available, for example from the company Hocoma AG under the trademark Lokomat^{®}. Such devices, based on the treadmill therapy are described in detail in US 6,821,233 and the prior art described therein, which is fully incorporated by reference herein.

Thus, said automated movement therapy can be carried out by using a device comprising a driven and controlled orthotetic device which guides the legs of said patient in a physiological pattern of movement, a treadmill and, preferably, a relief mechanism acting on the body weight of said patient. Preferably, such a device is used for treating gait movement disorders.

In particular, within the method of the present invention, use is made of the devices as claimed in US 6,821,233. One device is an apparatus for treadmill training, comprising a treadmill, a relief mechanism for the patient, and a driven orthotic device, wherein a parallelogram fixed in a height-adjustable manner on the treadmill is provided for stabilizing the othotic device and preventing the patient from tipping forward, backwards and sidewards, the parallelogram being attached to the orthotic device. The orthotic device preferably comprises a hip orthotic device and two leg parts, whereby two hip drives are provided for moving the hip orthotic device, and two knee drives are provided for moving the leg parts; the hip orthotic device and leg parts are adjustable, the leg parts are provided with cuffs which are adjustable in size and position; and a control unit is provided for controlling the movements of the orthotic device and controlling the speed of the treadmill.

Alternatively, another device is an apparatus for treadmill training, comprising a treadmill including a railing, a relief mechanism for the patient, and a driven orthotic device, wherein means for stabilizing the orthotic device are provided that prevent the patient from tipping forward, backwards and sidewards. The orthotic device preferably comprises a hip orthotic device and two leg parts, two hip drives are provided for moving the hip orthotic device, and two knee drives are provided for moving the leg parts; a ball screw spindle drive is provided for each knee drive and hip drive, the orthotic device and leg parts are adjustable, the leg parts are provided with cuffs which are adjustable in size and position; a control unit is provided for controlling the movements of the orthotic device and controlling the speed of the treadmill can be used. Both devices are described in detail in US 6,821,233.

In these devices, the relief force is provided by attaching a roller above the base frame, over which roller a wire cable is passed that is attached near the bearing and is loaded on the other side of the parallelogram with a counterweight (see Fig. 1 of US 6,821,233). An improved device for adjusting the height of and the relief force acting on the weight of a patient is the subject of EP 1 586 291 A1, which is also incorporated herein by reference. The device for adjusting the height of and the relief force acting on the weight of the patient is characterized in that said weight is supported by a cable, with a first cable length adjustment means to provide an adjustment of the length of the cable to define the height of said suspended weight and a second cable length adjustment means to provide an adjustment of the length of the cable to define the relief force acting on the suspended weight.

In a further embodiment of the present invention, the movement therapy is carried out by using an apparatus for locomotion therapy for the rehabilitation of paraparetic and hemiparetic patients, comprising a standing table that is preferably adjustable in height and inclination, a fastening belt with holding devices on the standing table for the patient, a drive mechanism for the leg movement of the patient, consisting of a knee mechanism and a foot mechanism, wherein the standing table has a head portion displaceable with respect to a leg portion about a pivot joint, whereby the pivot joint provides an adjustable hip extension angle for which an adjustable mechanism is provided. Preferably, the knee portion and foot portion are displaceably arranged on rails on the leg mechanism. Preferably, the foot mechanism serves to establish force on the sole of the foot during knee extension. Preferably, a control unit is provided for controlling the movement of the apparatus. Further details regarding said apparatus and its method of operation can be derived from US 6,685,658, which is again incorporated herein by reference in full.

In another embodiment of the present invention, in regard to the movement therapy, use is made of a device for applying a force between first and second portions of an animate body, said device comprising: first and second link assemblies associated with said first and second portions, respectively, each of said first and second link assembly preferably comprising: (a) a supporting section secured in position on a portion, each supporting section being a supporting link; and (b) an articulated link attached through a joint to each of said supporting links; wherein said articulated links of said first and second link assemblies are attached to each other through a pivot joint, with said articulated link of said second assembly extending beyond said pivot joint; first and second casings attached to a link in said first link assembly; first and second tendons extending through said first and second casings, respectively, and attached to a link in said second link assembly, wherein one of said tendons is attached to said articulated link in said second link assembly on one side of said pivot joint and the other tendon is attached to said articulated link in said second link assembly on the opposite side of said pivot joint.

Alternatively, use is made of a device for applying a force between first and second portions of a hand, one of said portions being a phalanx, said device comprising: first and second link assemblies associated with said first and second portions, respectively; each link assembly preferably comprising: (a) a supporting section secured in position on a portion, each supporting section being a supporting link; and (b) an articulated link attached through a joint to each of said supporting links; wherein said articulated links of said first and second link assemblies are attached to each other through a pivot joint, with said articulated link of said second assembly extending beyond said pivot joint; first and second casings attached to a link in said first link assembly; and first and second tendons extending through said first and second casings, respectively, and attached to a link in said second link assembly, wherein one of said tendons is attached to said articulated link in said second link assembly on one side of said pivot joint and the other tendon is attached to said articulated link in said second assembly on the opposite side of said pivot joint. Further details as to the device as such and the method of its use may be derived from US 6,059,506, which is again incorporated herein by reference in full.

Within the scope of the present invention, any other commercially available device or any device as developed in academic facilities and suited for automated locomotion therapies may be used. In this respect, mention is made of the "MIT-Manus" device, the "Mirror-Image Motion Enabler" (MIME) robot, the "ARM guide", the "Bi-Manu-Track" arm trainer, the GTI, an electromechanical gait trainer, and the NeRobot and REHAROB devices. More details regarding these devices may be taken from Hesse S, Schmidt H, Werner C, Bardeleben A. (2003), Upper and lower extremity robotic devices for rehabilitation and for studying motor contro, Curr Opin Neurol 16: 705-710) and the literature cited therein.

More preferably, devices as commercially available from the company Hocoma AG are used, in particular those commercialized under the trademark Lokomat® and Armeo^{®} at the filing date of the present application.

As recited above, in accordance with the present invention, the automated movement therapy is used in combination with the administration of a pharmaceutical composition/medicament comprising an effective amount of a chemodenervating agent, preferably a botulinum toxin, to said patient, wherein the administration is carried out prior to and/or during and/or after the movement therapy.

Botulinum toxin is produced by the bacterium *Clostridium.* Seven antigenically distinct serotypes of botulinum toxin are presently known, namely botulinum toxin A, B, C, D, E, F and G. Botulinum toxins, when released from lysed *Clostridium* cultures are generally associated with other bacterial proteins, which together form of a toxin complex.

The neurotoxic subunit of this complex is referred herein as the "neurotoxic component" of the botulinum toxin complex.

The terms "botulinum toxin" or "botulinum toxins" as used throughout the present applicaton, refer to the neurotoxic component devoid of any other clostridial proteins, but also to the "botulinum toxin complex": The term "botulinum toxin" is used herein in cases when no discrimination between the complex or the neurotoxic component is necessary or desired. The complex usually contains additional, so-called "non-toxic" proteins, which we will refer to as "complexing proteins" or "bacterial proteins".

The complex of neurotoxic component and bacterial proteins is referred to as *"Clostridium botulinum* toxin complex" or *"botulinum* toxin complex". The molecular weight of this complex may vary from about 300,000 to about 900,000 Da. The complexing proteins are, for example, various hemagglutinins. The proteins of this toxin complex are not toxic themselves but provide stability to the neurotoxic component during passage through the gastrointestinal tract. Medicaments on the basis of the botulinum toxin complex of types A, B and C are commercially available, type A botulinum toxin from lpsen (under the trademark Dysport^{®}) and from Allergan Inc. under the trademark Botox^{®}.

The neurotoxic component of the botulinum toxin complex is initially formed as a single polypeptide chain, having, in the case of serotype A, a molecular weight of approximately 150 kDa. In other serotypes, the neurotoxic component has been observed to vary between about 145 and about 170 kDa, depending on the bacterial source.

In the case of serotype A, for example, proteolytic processing of the polypeptide results in an activated polypeptide in the form of a dichain polypeptide, consisting of a heavy chain and a light chain, which are linked by a disulfide bond. In humans, the heavy chain mediates binding to pre-synaptic cholinergic nerve terminals and internalization of the toxin into the cell. The light chain is believed to be responsible for the toxic effects, acting as zink-endopeptidase and cleaving specific proteins responsible for membrane fusion (SNARE complex) (see e.g. Montecucco C., Shiavo G., Rosetto O: The mechanism of action of tetanus and botulinum neurotoxins, Arch Toxicol. 1996; 18 (Suppl.): 342-354).

By disrupting the process of membrane fusion within the cells, botulinum toxins prevent the release of acetylcholine into the synaptic cleft. The overall effect of botulinum toxin at the neuro-muscular junction is to interrupt neuro-muscular transmission, and, in effect, denervate muscles. Botulinum toxin also has activity at other peripheral cholinergic synapses, causing a reduction of salivation or sweating.

Each serotype of botulinum toxin binds to the serotype specific receptor sites on the pre-synaptic nerve terminal. The specificity of botulinum toxin for cholinergic neurons is based on the high affinity of the heavy chain for the receptor sites on these nerve terminals (Ref.: Katsekas S., Gremminloh G., Pich E.M.: Nerve terminal proteins; to fuse to learn. Transneuro Science 1994; 17: 368-379).

A pharmaceutical composition comprising the neurotoxic component of botulinum toxin type A in isolated form is commercially available in Germany from Merz Pharmaceuticals GmbH under the trademark Xeomin^{®}.

Within this invention, all forms of botulinum toxin, in particular the various serotypes, the various complexes of the neurotoxic component of botulinum toxin and its complexing accompanying proteins and the neurotoxic component of these botulinum toxins are to be used. In addition thereto, modified and/or recombinantly produced botulinum toxins or neurotoxic components of botulinum toxins including the respective mutations, deletions, etc. are also within the scope of the present invention. With respect to suitable mutants, reference is made to WO 2006/027207 A1, which is fully incorporated by reference herein. Furthermore, within the present invention, mixtures of various serotypes (in the form of the complex, the neurotoxic component and/or recombinant form), e.g. mixtures of botulinum toxins of types A and B or mixtures of botulinum neurotoxins of types A and B are also to be used.

The production of the neurotoxic component of botulinum toxin type A and B are described, for example, in the international patent application WO 00/74703.

The neurotoxic component referred to herein above, may be part of a composition or a pharmaceutical composition. This pharmaceutical composition may contain the neurotoxic component as the sole active component or may contain additional pharmaceutically active components.

A "pharmaceutical composition" is a formulation in which an active ingredient for use as a medicament or a diagnostic is contained or comprised. Such pharmaceutical composition may be suitable for diagnostic or therapeutic administration (i.e. by intramuscular or subcutaneous injection) to a human patient. The pharmaceutical composition may be lyophilized or vacuum dried, reconstituted, or may prevail in solution. When reconstituted, it is preferred that the reconstituted solution is prepared adding sterile physiological saline (0.9% NaCl).

Such composition may comprise additional components such as a pH buffer, excipient, diluent, cryoprotectant and/or stabilizer.

A "pH buffer" refers to a chemical substance being capable to adjust the pH value of a composition, solution and the like to a certain value or to a certain pH range.

"Stabilizing", "stabilizes" or "stabilization" means that the active ingredient, i.e., the neurotoxic component in a reconstituted or aqueous solution pharmaceutical composition has greater than about 20%, 30%, 40%, 50%, 60%, 70%, 80%, 90%, and up to about 100% of the toxicity that the biologically active neurotoxic component had prior to being incorporated into the pharmaceutical composition.

"Cryoprotectant" refers to excipients which result in an active ingredient, i.e., a neurotoxic component in a reconstituted or aqueous solution pharmaceutical composition that has greater than about 20%, 30%, 40%, 50%, 60%, 70%, 80%, 90%, and up to about 100% of the toxicity that the biologically active neurotoxic component had prior to being freeze-dried in the pharmaceutical composition.

Examples of such stabilizers are gelatin or albumin, preferably of human origin or obtained from a recombinant source. Proteins from non-human or non-animal sources are also included. The stabilizers may be modified by chemical means or by recombinant genetics. In a preferred embodiment of the present invention, it is envisaged to use alcohols, e.g., inositol, mannitol, as cryoprotectant excipients to stabilize proteins during lyophilization.

In a more preferred embodiment of the present invention, the stabilizer may be a non proteinaceous stabilizing agent comprising hyaluronic acid or polyvinylpyrrolidone or polyethyleneglycol or a mixture of two or more thereof. Such composition is considered to be a safer composition possessing remarkable stability.

The pharmaceutical composition to be used herein may comprise botulinum toxin, e.g. in the form of the neurotoxic component and a hyaluronic acid or a polyvinylpyrrolidone or a polyethleneglycol, such composition being optionally pH stabilized by a suitable pH buffer, in particular by a sodium acetate buffer, and/or a cryoprotectant polyalcohol.

Irrespective of whether the pharmaceutical composition comprises, besides the neurotoxin component, additional components such as the complexing proteins of the Botulinum toxin complex, albumin, hyaluronic acid, a polyvinylpyrrolidone and/or a polyethyleneglycol stabilizer, the pharmaceutical composition in accordance with the present invention preferably retains its potency substantially unchanged for six month, one year, two year, three year and/or four year periods when stored at a temperature between about +8°C and about -20°C. Additionally, the indicated pharmaceutical compositions may have a potency or percent recovery of between about 20% and about 100% upon reconstitution.

The pharmaceutical composition as used in the present invention may include the neurotoxic component, and, optionally, one or more additional components. Preferably, the pharmaceutical composition has a pH of between about 4 and 7.5 when reconstituted or upon injection, more preferably between about pH 6.8 and pH 7.6 and most preferably between pH 7.4 and pH 7.6. Generally, the pharmaceutical composition of the present invention comprises neurotoxic component in a quantity of about 6 pg to about 30 ng, Preferably, the neurotoxic component has a biological activity of 50 to 250 LD₅₀ units per ng neurotoxic component, as determined in a mouse LD₅₀ assay. More preferably, the neurotoxic component has a biological activity of about 150 LD₅₀.

The pharmaceutical composition used in the present invention may comprise a neurotoxin, and a hyaluronic acid. The hyaluronic acid preferably stabilizes the neurotoxin. The pharmaceutical compositions disclosed herein may have a pH of between about 4 and 7.5 when reconstituted or upon injection. The hyaluronic acid in the instant pharmaceutical composition is preferably combined with the instant neurotoxic component in a quantity of 0.1 to 10 mg, especially 1 mg hyaluronic acid per ml in a 200 U/ml botulinum toxin solution. More preferably, the subject solution also contains a 1-100 mM, preferably 10 mM sodium acetate buffer.

In another preferred embodiment, the composition may contain a polyalcohol as cryoprotectant. Examples of polyalcohols are preferably used include inositol, mannitol or other non-reducing alcohols.

In particular, those embodiments of the pharmaceutical composition of the present invention that do not comprise a proteinaceous stabilizer, preferably also do not contain trehalose or maltotriose or related sugar or polyhydroxy compounds which are sometimes used as cryoprotectants.

The polyvinylpyrrolidone in the instant pharmaceutical composition is preferably combined with the instant neurotoxic component in a quantity of 10 to 500 mg, especially 100 mg polyvinylpyrrolidone per ml in a 200 U/ml botulinum toxin solution. More preferably, the subject solution also contains a 1-100 mM, preferably 10 mM sodium acetate buffer.

The polyethyleneglycol in the instant pharmaceutical composition is preferably combined with the instant neurotoxic component in a quantity of 10 to 500 mg, especially 100 mg polyethyleneglycol per ml in a 200 U/ml botulinum toxin solution. More preferably, the subject solution also contains a 1-100 mM, preferably 10 mM sodium acetate buffer.

Within the present patent application, the use of a medicament based on the neurotoxic component of botulinum toxin type A, and more particular the product distributed by Merz Pharmaceutical under the trademark Xeomin^{®} is preferred. This is because the tendency of generating antibodies within the patient was found to be lower when applying pharmaceutical compositions on the basis of the neurotoxic component of botulinum toxin, such as Xeomin^{®} compared to administering medicaments on the basis of the botulinum toxin type A complex. Without being bound to any theory, it is believed that the hemagglutinins within the botulinum toxin complex have an activating capability on the immune system.

As indicated above, the pharmaceutical composition comprising the botulinum toxin is administered, preferably several times, in an effective amount for improving the patient's condition either prior to and/or during and/or after said locomotion therapy. Preferably, an effective amount of botulinum toxin is administered several times during the movement therapy, and the composition is administered for the first time before commencement of any movement/locomotion therapy.

Typically, the dose administered to the patient will be up to about 1000 units, but in general should not exceed 400 units per patient. The most preferable range lies between about 80 to about 400 units. These values are preferably valid for adult patients. For children, the respective doses range from 25 to 800 and preferably from 50 to 400 units.

While the above ranges relate to the maximum total doses, the dose range per muscle is preferably within 3 to 6 units/kg body weight (b.w.), for small muscles 0,5-2 U/kg b.w., preferably 0,1-1 U/kg b.w. Generally doses should not exceed 50 U per injection site and 100 U per muscle.

In one embodiment of the present invention the effective amount of botulinum toxin administered exceeds 500 U of neurotoxic component in adults or exceeds 15 U/kg body weight in children.

As to the frequency of dosing, the re-injection interval is preferably greater than 3 months. This is particularly true when applying medicaments on the basis of the botulinum toxin complex, where there exists an increased likelihood for the occurrence of antibodies.

When applying medicaments on the basis of the neurotoxic component of botulinum toxin, such as Xeromin^{®}, a more frequent dosing is also possible. Thus, according to the present invention the medicament to be administered is re-administered in intervals of between 3 and 6 months, in another embodiment, particularly when using the neurotoxic component of botulinum toxin, more preferably Xeomin®, the medicament is re-administered in intervals of between 2 weeks and less than 3 months. It is, however, most preferred to re-administer the medicament at a point in time when muscular activity (again) interferes with the automated movement therapy.

With regard to the composition and dosing of the medicament on the basis of botulinum toxin, and in regard to the composition, dosing and frequency of administration of the medicament on the basis of the neurotoxic component of botulinum toxin, reference is made to US 60/817 756 incorporated herein by reference.

While the above stated values are to be understood as a general guideline for administering the medicament as used within the present invention, it is, however, ultimately the physician who is responsible for the treatment who decides on both the quantity of toxin administered and the frequency of its administration. In the present method, the medicament on the basis of botulinum toxin is preferably re-administered at a point in time at which the movement ability of the patient is (again) deteriorating compared to the movement ability at the point of maximum therapeutic effect of botulinum toxin.

The medicament on the basis of botulinum toxin can be to be injected directly into the affected muscles. In order to find the appropriate injection site, several means exist which help the physician in order to find the same. Within the present invention, all methods for finding the best injection site are applicable, such as injection guided by electromyography (EMG), injection guided by palpation, injection guided by CT/MRI, as well as injection guided by sonography. Among those methods, the latter is preferable and is the method of choice when treating children. With respect to further details regarding the injection guided by sonography, we refer to Berweck "Sonography-guided injection of botulinum toxin A in children with cerebral palsy", Neuropediatric 2002 (33), 221-223.

Furthermore, the invention relates to kit for the treatment of patients suffering from movement disorders comprising a medicament comprising an effective amount of a chemodenervating agent, and a device for carrying out automated movement therapy.

Preferably, the device for carrying out automated movement therapy within said kit comprises a driven and controlled gait orthosis and/or arm mover which guides the extremities of said patient in a physiological pattern of movement. More preferably it is one of the devices described in detail hereinbefore. Within the kit, the medicament preferably comprises a botulinum toxin as the chemodenervating agent, more preferably the device is a medicament on the basis of the neurotoxic component of Botulinum toxin A.

Within said kit, the medicament is specifically adapted to be used in combination with locomotion therapy, preferably in combination with the respective device that is used for said therapy. Such specific adaptation, which may be carried out specifically in relation to the kit according to the present invention but also within the medicament commercialized as such can be achieved by way of a specifically adapted packaging and/or the packaging leaflet and/or instructions of use of the medicament to be used within the present invention.

With respect to the above, it is to be understood that the disclosure of all prior art (pre-published and non-prepublished) as recited above is incorporated herein by reference in full.

Hereinafter, the present invention is illustrated by way of non-limiting examples.

### EXAMPLES

### Example 1

A study was carried out with 9 patients in the department of Pediatric Neurology and Developmental Medicine of the Dr. von Haunersches Children's Hospital Munich (Germany). Approval for the studies was obtained from local ethics committees.

Patients were eligible for our study if they had central gait impairment due to either congenital or acquired brain or spinal lesions. Femur length had to be at least 21 cm, which applies to children of an age of approximately 4 years. Achieving walking ability had to be a realistic goal of the rehabilitation program. Patients had to be able to reliably signal pain, fear or discomfort. Written informed consent of the parents was a prerequisite.

Exclusion criteria were: Severe lower-extremity contractures, fractures, osseous instabilities, osteoporosis, contraindication of full body load due to operations, severe dysproportional bone growth, unhealed skin lesions in the lower-extremity, thromboembolic diseases, cardio-vascular instability, acute or progressive neurological disorders and aggressive or self-harming behaviour.

Eight children and one adolescent were referred to our outpatient clinic for participated in the Driven Gait Orthosis (DGO) training program between January and August 2006. Mean age at the beginning of the training was 8y 2mo (SD2y 10mo, range 5y 2mo-14y 4mo). All but one patient trained on the children's module of the DGO (Table 2).

The automated locomotor training was performed by the commercially available DGO Lokomat^{®} (Hocoma AG, Volketswil, Switzerland). The adult - as well as the new pediatric version of the DGO were used. The DGO consists of two leg orthoses which are adjustable to the anatomy of different patients. It is fastened to the legs by several braces. The width of the hip orthosis, the length of the upper and lower leg as well as the size and position of the leg braces can be varied. The main difference between the adult (femur length > 350mm) and pediatric module (210mm to 350mm) is in the length of the thigh. The DGO is connected to the frame of a body weight support system by a four bar linkage. This allows movement of the orthosis in a vertical direction and provides additional vertical stability. On each leg, two linear drives move the hip and the knee joint of the orthosis. These drives are controlled by a position controller (real-time system implemented on a PC) that conducts a kinematic pattern resembling normal walking. The movements of the DGO are synchronised with the treadmill. The walking speed can be set between 1 and 3.2km/h. To ensure safe training, several safety features are integrated into the system. These include stop buttons for the therapists and the patients and a controller that limits both excessive forces at the drives and deviations from the desired position in the joint angles. The dorsiflexion of the ankle joint is provided by an elastic foot strap. To ensure a most physiological gait pattern and to prevent the skin from excoriation, proper fixation of the patient to the DGO is of utmost importance. For body weight support, a counter weight system is used. This allows body weight support within a range of 5 to 80kg in 5kg steps.

The amount of unloading was set at 50 percent of body weight initially, to be decreased successively according to the gain of muscular strength (allowing no excessive knee flexion during stance). The initial gait velocity for the training was chosen according to the capabilities of the child.

Training on the DGO included three to four sessions of 25-45 minutes per week. 10 to 13 sessions were conducted. Nearly all of the patients stopped their usual weekly physiotherapy sessions because of time limits. Eight out of 10 patients were referred for Botox® treatment of muscles of the lower extremity (Table 2).

To assess the feasibility of the DGO training, walking time, covered distance and gait speed of each session were logged. Motor performance tests were assessed before and at the end of the training program. Gait speed was assessed with the 10 Meter Walking Test (10MWT). Children were instructed to walk at their comfortable speed.

To assess changes in motor functions, the standing (dimension D) and walking sections (dimension E) of the GMFM-66 were administered by a GMFM (Gross Motor Function Measure)-certificated therapist. Additionally, children of the inpatient group performed a 6-Min Walking Test (6MWT) to evaluate gait endurance. To determine the amount of assistance the child requires during walking, the Functional Ambulation Categories (FAC) were used. All tests were accomplished using the same assistive devices before and after the intervention.

Parameters were checked in regard to their respective normal distribution. This applied to the results of gait speed and 6MWT. Thus differences between pre- and posttraining were analyzed using the repeated t-test. All other parameters were analyzed with the nonparametric Wilcoxon signed rank test.

Eight of the nine patients completed the training on the DGO. One patient dropped out after the third training due to reduced compliance. The total number of training sessions on the DGO was 12 (SD 1.0, range 10-13). The participants walked on average 1158m (SD 371m, range 410-1675m) per session. Mean training duration was 28:42 minutes (SD 3:30 minutes, range 23-32 minutes). The average walking speed was 1.7km/h (SD 0.17 km/h, range 1.5-2.1km/h) with unloading of 14.4% (SD 12.6% of body weight, range 0-30%) (figure 2c,d).

Over-ground walking parameters were assessed in seven patients and improved in all of them. Mean gait speed increased significantly from 0.87m/s (SD 0.32m/s) to 1.09m/s (SD 0.31m/s, T=-3.11, *p*=0.01) (figure 3e). Seven of the eight patients showed an improvement in dimension D and E. The mean score of dimension D changed markedly from 46.7 (SD 34.1) to 52.0 (SD 28.8), although statistical analysis revealed no significance (Z=-1.820, p=0.69,). In dimension E the mean score increased slightly but significantly from 39.5 (SD 32.6) to 42.2 (SD 34.6) after training (Z=-2,366, p<0.05). (figure 3f+g) Walking abilities, as assessed with FAC, showed no changes.

Gait speed and the GMFM score improved significantly. Results of the walking section in the GMFM revealed significant improvements in contrast to the less distinct findings in the standing section. An overview of the study is given in Table 2 below.

**Table 2:**

| **Patient No.** | **Sex** | **Age (y:m)** | **Diagnosis** | **Lokomat-Type** | **Number of trainings** | **Total walking distance (km)** | **BoNT/A Treatment before** |
|---|---|---|---|---|---|---|---|
| 1 | F | 6:4 | CP (I) | CM | 13 | 17.2 | Y |
| 2 | F | 9:10 | CP (II) | CM | 12 | 13.6 | Y |
| 3 | F | 10:10 | CP (II) | CM | 13 | 18.6 | N |
| 4 | M | 6:2 | CP (III) | CM | 10 | 9.9 | Y |
| 5 | M | 6:4 | CP (III) | CM | 12 | 13.3 | Y |
| 6 | M | 14:4 | CP (II) | AM | 12 | 12.5 | Y |
| 7 | F | 7:0 | CP (IV) | CM | 3 | 0.9 | Y |
| 8 | M | 8:0 | CP (IV) | CM | 10 | 5,0 | N / Y |
| 9 | M | 5:2 | CP (III) | CM | 10 | 6,7 | Y |

### Example 2

An 8 year old male patient with a bilateral spastic cerebral palsy due to prematurity associated brain damage was subjected to 12 sessions of Robotic assisted treadmill training using the Pediatric Lokomat®. The patient was not able to perform more than 2 training session as the internal control of the robotic device stopped the training due to elevated resistance related to increased muscle tone. Botulinum toxin treatment (total does 15U/ kg KG Botox®) was done in a multilevel approach of the lower extremity including hip flexors, knee flexors, adductor muscles and gastrocnemius muscle. After this intervention the Robotic assisted treadmill training was easily continued and the patient was able to perform all suggested 12 sessions. After these interventions there were significant improvements of endurance (from 344-751 m in the 6 min running test) and motor function (from 2,5% to 10% in walking dimension of the Gross Motor Function measure).

This example shows the synergetic effect of combining the administration of a denervating agent such as botulinum toxin leading to an at least temporary relief of muscle spasticity with movement therapy that can only effectively work on an at least partially relaxed muscle. A muscle then strengthened or conditioned by the movement therapy may be exposed to a potentially higher dose of denervating agent, thus leading to further synergetic effects.

## Claims

1. Use of an effective amount of a chemodenervating agent for the manufacture of a medicament for administering to a patient for treating a movement disorder in said patient, wherein said patient is subjected to an automated movement therapy, and wherein said medicament is administered prior to and/or during and/or after said movement therapy.

2. Use according to claim 1, wherein the movement disorder is a hyperkinetic and/or hypokinetic movement disorder, wherein an inmbalance between agonist and antagonist is interfering with function.

3. Use according to claim 1 or claim 2, wherein said automated movement therapy is supported by an automated gait orthosis or an arm mover.

4. Use according to claim 3, wherein said automated gait orthosis is used in combination with a treadmill.

5. Use according to claim 1, wherein said automated movement therapy is carried out by using a device comprising a driven and controlled orthotetic device which guides the legs of said patient in a physiological pattern of movement, preferably further using a treadmill and a relief mechanism acting on the body weight of said patient.

6. Use according to claim 5, wherein the relief mechanism comprises means for adjusting the height of and the relief force acting on the weight of the patient, wherein said weight is supported by a cable, with a first cable length adjustment means to provide an adjustment of the length of the cable to define the height of said suspended weight and a second cable length adjustment means to provide an adjustment of the length of the cable to define the relief force acting on the suspended weight.

7. Use according to claim 5 or 6, wherein the automated movement therapy is carried out by employing an apparatus for treadmill training, comprising a treadmill, a relief mechanism for the patient, and a driven orthotic device, wherein a parallelogram fixed in a height-adjustable manner on the treadmill is provided for stabilizing the orthotic device and preventing the patient from tipping forward, backwards and sidewards, the parallelogram being attached to the orthotic device; the orthotic device comprises a hip orthotic device and two leg parts, whereby two hip drives are provided for moving the hip orthotic device, and two knee drives are provided for moving the leg parts; the hip orthotic device and leg parts are adjustable, the leg parts are provided with cuffs which are adjustable in size and position; a control unit is provided for controlling the movements of the orthotic device and controlling the speed of the treadmill.

8. Use according to claim 5 or 6, wherein the automated movement therapy is carried out by employing an apparatus for treadmill training, comprising a treadmill including a railing, a relief mechanism for the patient, and a driven orthotic device, wherein means for stabilizing the orthotic device are provided that prevent the patient from tipping forward, backward and sideward; the orthotic device comprises a hip orthotic device and two leg parts, two hip drives are provided for moving the hip orthotic device, and two knee drives are provided for moving the leg parts; a ball screw spindle drive is provided for each knee drive and hip drive, the orthotic device and leg parts are adjustable, the leg parts are provided with cuffs which are adjustable in size and position; a control unit is provided for controlling the movements of the orthotic device and controlling the speed of the treadmill.

9. Use according to claim 1, wherein the automated movement therapy is carried out by employing an apparatus for locomotion therapy for the rehabilitation or habilitation of bilateral or unilateral spastic conditions in paraparetic and hemiparetic patients, comprising a standing table adjustable in height and inclination, a fastening belt with holding devices on the standing table for the patient, a drive mechanism for the leg movement of the patient, consisting of a knee mechanism and a foot mechanism, wherein the standing table has a head portion displaceable with respect to a leg portion about a pivot joint, whereby the pivot joint provides an adjustable hip extension angle for which an adjusting mechanism is provided; and the knee portion and foot portion are displaceably arranged on rails on the leg mechanism; the foot mechanism serves to establish force on the sole of the foot during knee extension; a control unit is provided for controlling movement of the apparatus.

10. Use according to any one of claims 1 to 9, wherein the automated movement therapy is carried out by employing a device for applying a force between first and second portions of an animate body, said device comprising: first and second link assemblies associated with said first and second portions, respectively, each said first and second link assembly comprising:
a) a supporting section secured in position on a portion, each supporting section being a supporting link; and
b) an articulated link attached through a joint to each of said supporting links; wherein said articulated links of said first and second link assemblies are attached to each other through a pivot joint, with said articulated link of said second assembly extending beyond said pivot joint; first and second casings attached to a link in said first link assembly; first and second tendons extending through said first and second casings, respectively, and attached to a link in said second link assembly, wherein one of said tendons is attached to said articulated link in said second link assembly on one side of said pivot joint and the other tendon is attached to said articulated link in said second link assembly on the opposite side of said pivot joint.

11. Use according to any one of claims 1 to 9, wherein the automated movement therapy is carried out by employing a device for applying a force between first and second portions of a hand, one of said portions being a phalanx, said device comprising: first and second link assemblies associated with said first and second portions, respectively, each link assembly comprising:
a) a supporting section secured in position on a portion, each supporting section being a supporting link; and
b) an articulated link attached through a joint to each of said supporting links; wherein said articulated links of said first and second link assemblies are attached to each other through a pivot joint, with said articulated link of said second assembly extending beyond said pivot joint; first and second casings attached to a link in said first link assembly; and first and second tendons extending through said first and second casings, respectively, and attached to a link in said second link assembly, wherein one of said tendons is attached to said articulated link in said second link assembly on one side of said pivot joint and the other tendon is attached to said articulated link in said second assembly on the opposite side of said pivot joint.

12. Use according to any one of claims 1 to 11, wherein the movement disorder is associated with cerebral palsy, M. Parkinson, central gait impairment, spinal cord injuries, dystonias, traumatic brain injury, genetic disorders, metabolic disorders, dynamic muscle contractures and/or stroke.

13. Use according to claim 12, wherein the movement disorder is associated with at least one selected among pes equinus, pes varus, lower limb spasticity, upper limb spasticity, adductor spasticity, hip flexion contracture, hip adduction, knee flexion spasticity (crouch gait), plantar flexion of the ankle, supination and pronation of the subtalar joint, writer's cramp, musician's cramp, golfer's cramp, leg dystonia, thigh adduction, thigh abduction, knee flexion, knee extention, equinovarus deformity, foot dystonia, striatal toe, toe flexion, toe extension.

14. Use according to any one of claims 1 to 13, wherein the patient is human.

15. Use according to claim 14, wherein the patient has not completed its motor development and fixed muscle contractures have not occurred.

16. Use according to claim 15, wherein the patient is a child up to six years in age.

17. Use according to any one of claims 1 to 16, wherein the chemodenervating agent is a botulinum toxin.

18. Use according to claim 1 or 17, wherein said medicament is administered by injection.

19. Use according to claim 1 or 18, wherein said medicament is administered several times during the treatment.

20. Use according to claim 19, wherein said medicament is administered for the first time before commencement of the movement therapy.

21. Use according to claim 19, wherein said medicament is re-administered in intervals of between 3 and 6 months.

22. Use according to claim 19, wherein said medicament is re-administered in intervals of between 2 weeks and less than 3 months.

23. Use according to claim 19, wherein said composition is re-administered at a point in time when muscular activity interferes with the automated movement therapy.

24. Use according to claim 17, wherein the effective amount of botulinum toxin administered exceeds 500 U of neurotoxic component in adults or exceeds 15 U/kg body weight in children.

25. Use according to claim 17, wherein the botulinum toxin is the botulinum toxin complex type A.

26. Use according to claim 17, wherein the botulinum toxin is the neurotoxic component of a *Clostridium botulinum* toxin complex.

27. Use of claim 17 or 26, wherein the botulinum toxin is selected from the group consisting of serotypes A, B, C, D, E, F, G and a mixture thereof.

28. Use of claim 26, wherein the neurotoxic component is of type A.

29. A kit for the treatment of patients suffering from movement disorders comprising
a) a medicament comprising an effective amount of a chemodenervating agent; and
b) a device for carrying out automated movement therapy.

30. Kit according to claim 29, wherein the device for carrying out automated movement therapy comprises a driven and controlled gait orthosis and/or arm mover which guides the extremities of said patient in a physiological pattern of movement.

31. Kit according to claim 29, wherein the chemodenervating agent is a botulinum toxin.

32. Kit according to claim 31, wherein the botulinum toxin is the neurotoxic component of a *Clostridium botulinum* toxin complex.
